# EUROPEAN PATENT APPLICATION

(11) **EP 1 950 310 A1**
(43) Date of publication of application: **30.07.2008**
(21) Application number: 07001409.7
(22) Date of filing: 23.01.2007
(51) Int. Cl.: C12Q 1/68, G01N 33/68

(54) **Method for risk prediction of a postoperative sepsis in a human**

(71) Applicant: Charité-Universitätsmedizin Berlin, 10117 Berlin (DE)
(72) Inventor: Kotsch, Katja, 13503 Berlin (DE); Volk, Hans-Dieter, 10117 Berlin (DE); Keh, Didier, 14050 Berlin (DE)
(74) Representative: Krauss, Jan

(57) **Abstract**

The present invention generally relates to the field of diagnosis, and in particular to a method suitable to predict the risk of a postoperative sepsis in a human. The present invention further relates to a diagnostic kit for the risk prediction of a postoperative sepsis in a human.

## Description

The present invention generally relates to the field of diagnosis, and in particular to a method suitable to predict the risk of a postoperative sepsis in a human. The present invention further relates to a diagnostic kit for the risk prediction of a postoperative sepsis in a human.

### BACKGROUND OF THE INVENTION

Despite greatly improved diagnosis, treatment and support, serious infection and sepsis remain significant causes of death and often result in chronic ill-health or disability in those who survive acute episodes. For instance, sepsis is one of the most common causes of death in Germany. The incidence reaches 226 cases per 100.000 persons and year. The 90 day-lethality of a so-called "severe sepsis" is about 54% (Brunkhorst FM et al., Epidemiology, economy and practice - results of the German study on prevalence by the competence network sepsis (SepNet) Anasthesiol. Intensivmed. Notfallmed. Schmerzther. 2006; 41(1):43-4).

"Sepsis" is a serious medical condition, which results from a dysregulation of the host defence against microorganisms or microbial products. The typical immunologic features are hyperinflammation and immunodepression. Further, sepsis is defined as a systemic inflammatory response syndrome (SIRS) with the clinical proof of an infection. "Infection" is defined as a pathological process caused by invasion of a normally sterile tissue, fluid or body cavity by pathogenic or potentially pathogenic microorganisms. The clinical SIRS criteria are hyper- or hypothermia, leucocytosis or leukopenia, tachycardia, tachypnoe and hypercania.

More critical forms of sepsis are "severe sepsis" and "septic shock". "Severe sepsis" is defined as a sepsis which is complicated by an acute organ dysfunction, further defined by Marshall et al (Marshall JC et al., Multiple organ dysfunction score: a reliable descriptor of a complex clinical outcome. 1995, Crit Care Med 23: 1638-1652) or the sequential organ failure assessment (SOFA) score (Ferreira FL et al., Serial evaluation of the SOFA score to pre-dict outcome in critically ill patients 2001, JAMA 286 (14): 1754-1758). In adults, a "septic shock" refers to sepsis plus a state of acute circulatory failure characterised by a persistent arterial hypotension unexplainable by other causes.

The ability to detect potentially serious infections as early as possible and, especially, to predict the onset of sepsis in susceptible individuals is clearly advantageous. Many attempts have been made to design robust predictive models based on measuring a range of clinical, chemical, biochemical, immunological and cytometric parameters and a number of scoring systems, of varying prognostic success and sophistication, proposed.

For instance, according to the 1991 Consensus Conference of the American College of Chest Physicians (ACCP) and Society of Critical Care Medicine (SCCM) "SIRS" is considered to be present when patients show more than two parameters of the following: a body temperature of greater than 38°C or less than 36°C, a heart rate of greater than 90/min, hyperventilation involving a respiratory rate higher than 20/min or, blood gas Pa of CO₂ lower than 32mm Hg, a white blood cell count of greater than 12,000 cells/µl or less than 4,000 cells/µl (American College of Chest Physicians/Society of Critical Care Medicine Consensus Conference: definitions for sepsis and organ failure and guidelines for the use of innovative therapies in sepsis. 1992, Crit Care Med 20: 864-874).

In order to evaluate the seriousness of sepsis the correlation of sepsis and a number of specific serum markers has been extensively studied with a view to developing specific diagnostic and prognostic tests.

Following infection with infectious microorganisms, the body reacts with a classical inflammatory response and activation of, first, the innate, non-specific immune response, followed by a specific, acquired immune response. In the case of bacterial infections, bacteraemia leads to the rapid 20 (within 30-90 minutes) onset of pyrexia and release of inflammatory cytokines such as interleukin-1 (IL-1) and tumour necrosis factor-α (TNFα) triggered by the detection of bacterial toxins, long before the development of a specific, antigen-driven immune response.

The international patent application WO 2006/061644 describes a system and a method for detecting early signs of an infection and, in particular, for identifying individuals most likely to develop sepsis. Measurement of expression levels of particular combinations of cytokines and/or cellular activation markers, optionally combined with the use of predictive algorithms, allows a high degree of accuracy of prediction. The disclosed system measures, for instance, the increased levels of expression of TNFα and IL-1.

TNFα and IL-1 are archetypal acute inflammatory cytokines long known to be elevated in sepsis (Damas P et al., Tumor necrosis factor and interleukin-1 serum levels during severe sepsis in humans. 1989, Crit Care Med 17: 975-978). They have been reported to be useful predictors of organ failure in adult respiratory distress syndrome, a serious complication of sepsis (Meduni et al., 1995, Chest 107: 1062-1073). In addition, it has been reported that serum TNFα were significantly higher in patients with sepsis as a complication than in those without sepsis (Nakae H et al., Involvement of IL-18 and soluble fas in patients with postoperative hepatic failure. 2003, Eur Surg Res. 35(2): 61-6).

US patent 5,830,679 discloses a method of diagnosing sepsis in a human infant. The method includes detecting an increase in the expression of leukocyte cell surface antigens in a blood sample from an infant at risk for developing sepsis.

US patent 6,251,598 discloses methods and kits for detecting polymorphism that are predictive of a subject's susceptibility to developing sepsis, which involves detecting an allele of interleukin-1 genetic pattern that leads to dysregulated inflammatory response.

International patent application WO 03/102586 discloses a method for the early detection and detection, the progression prognosis and the evaluation of the degree of severity, and the treatment-accompanying progression evaluation of sepsis and sepsis-like systemic infections, and for the estimation of the danger which would be presented to patients at high risk of sepsis, by the development of a sepsis. According to the disclosed method, the presence and/or quantity of anti-asialo-GM1 antibodies (anti-AGM1 antibodies), and antibodies which cross-react with the same, in a biological liquid of a patient or a patient at high risk of sepsis is determined, and conclusions are drawn from the presence and/or quantity of the same in terms of presence, expected progression, degree of severity or the success of a treatment for the inflammatory disease or sepsis, or in terms of the danger presented to a patient at high risk of sepsis.

EP patent application 1 295 127 discloses the use of inter-alpha trypsin inhibitors as markers for diagnosis or prognosis of sepsis in a mammal, comprising contacting body fluid from a mammal with a ligand that binds the inhibitor, to form a complex that is detected.

RU patent application 2187809 discloses a method which provides the prediction at the earlier stages of abdominal sepsis development on the basis of evaluating the activity of Willebrand's factor as a marker of endothelial injure.

US 6,423,505 teaches that measuring HLA-DR/CD11b expression for determining suitability of immune therapy to sepsis involves detecting binding of antibody specific for HLA-DR/CD 11b after contacting with lysomotropic amine and the antibody. In addition, it has been shown that down-regulation of monocyte HLA-DR expression is a predictor of a poor outcome in sepsis and may be an indication of monocyte deactivation, impairing TNFα production. Treatment with IFN-y has been shown to be beneficial in such cases (Docke WD et al., Monocyte deactivation in septic patients: restoration by IFN-gamma treatment. 1997, Nature Med 3: 678-681).

US 2004/0096917 discloses that the early prediction or diagnosis of sepsis advantageously allows for clinical intervention before the disease rapidly progresses beyond initial stages to the more severe stages, such as severe sepsis or septic shock, which are associated with high mortality. In this application the early prediction or diagnosis is accomplished by comparing an individual's profile of biomarker expression to profiles obtained from one or more control, or reference, populations, which may include a population that develops sepsis. Recognition of features in the individual's biomarker profile that are characteristic of the onset of sepsis allows a clinician to diagnose the onset of sepsis from a bodily fluid isolated from the individual at a single point in time. The necessity of monitoring the patient over a period of time is, therefore, avoided, advantageously allowing clinical intervention before the onset of serious symptoms of sepsis. Further, because the biomarker expression is assayed for its profile, identification of the particular biomarkers is unnecessary. The comparison of an individual's biomarker profile to biomarker profiles of appropriate reference populations likewise can be used to diagnose SIRS in the individual.

US patent application 2006/0177848 provides a method of identifying a subject having a reduced risk of developing sepsis, comprising detecting at least one APOE3 allele in nucleic acids from the subject.

US 5,389,522 discloses serum antioxidants as predictors of the adult respiratory distress syndrome (ARDS) in septic patients. According to this disclosure, at the initial diagnosis of sepsis (6-24 h before the development of ARDS), serum manganese superoxide dismutase (MnSOD) levels and catalase activities are increased in septic patients who subsequently developed ARDS compared to septic patients who did not develop ARDS. Thus, increases in MnSOD and catalase may be used to predict the occurrence of ARDS in septic patients with the same sensitivity, specificity and efficiency as parallel assessments of serum lactate dehydrogenase (LDH) and Factor VIII levels. Evaluation of serum MnSOD and catalase as well as these other accessible markers are described as facilitating the identification of subsets of patients and allows prospective treatment of septic patients who are destined to develop ARDS.

After a major surgery, most patients suffer from an immunodepression. Such a postoperative immunodepression is one of the most important risk factor for a bacterial infection and for the developing of a postoperative sepsis. (Strohmeyer JC et al., Standardized immune monitoring for the prediction of infections after cardiopulmonary bypass surgery in risk patients. Cytometry B Clin Cytom. 2003 May; 53(1): 54-62). As mentioned above, the typical immunologic features of a sepsis are hyperinflammation and immunodepression. However, a post-operative sepsis presumably arises from an immunodepression, and not from a hyperinflammation. Therefore, although many of the above-mentioned methods might indications regarding the seriousness of the condition of a human in general, they are not suitable in order to diagnose a postoperative sepsis.

So far, it has been reported that procalcitonin represents a good biological diagnostic marker for sepsis, severe sepsis, or septic shock. Therefore, it has been suggested that procalcitonin should be included in diagnostic guidelines for sepsis (Uzzan B et al., Procalcitonin as a diagnostic test for sepsis in critically ill adults and after surgery or trauma: a systematic review and meta-analysis. Crit Care Med. 2006, 34(7): 1996-2003), Thuemer O et al., Procalcitonin as an early marker of sepsis. Anaesthesist. 2006; 55(6): 650-4). However, it has been also reported that procalcitonin does not discriminate between infectious and non-infectious complications (Dorge H et al., Procalcitonin is a valuable prognostic marker in cardiac surgery but not specific for infection. Thorac Cardiovasc Surg. 2003; 51(6): 322-6). Thus, procalcitonin is not a reliable marker to diagnose the development of a postoperative sepsis.

De Mendonca-Filho HT et al. (de Mendonca-Filho HT et al., Macrophage migration inhibitory factor is associated with positive cultures in patients with sepsis after cardiac surgery. Shock. 2005; 24(4): 313-7) analyzed the blood levels of macrophage migration inhibitory factor (MIF), other cytokines, and markers of acute-phase response in patients who developed the clinical syndrome of sepsis after cardiac surgery. In this study, no significant difference in the levels of C-reactive protein, procalcitonin, IL-6, IL-10, MCP-1, or TNFα between patients with positive cultures, having a higher mortality and higher levels of MIF than those with negative cultures was observed. In conclusion, circulating levels of MIF could be indicated as a valuable marker of microbiologically documented sepsis in patients after cardiac surgery.

In a study concerning a postoperative hepatic failure (Nakae H et al., Involvement of IL-18 and soluble fas in patients with postoperative hepatic failure. Eur Surg Res. 2003; 35(2): 61-6), it could be shown that the serum TNFα, IL-18, and sFas levels were significantly higher in patients with sepsis as a complication than in those without sepsis. The TNFα and IL-18 levels were significantly higher in non-survivors than in survivors. In addition, significant correlations were observed between TNFα and IL-6, between TNFα and IL-18, and between TNFα and sFas levels.

In a further study, the relationship between peaks of G-CSF serum concentrations and respiratory burst activity of polymorphonuclear cells (PMN) was investigated in patients with post-operative or post-traumatic severe sepsis and septic shock. Over a 12 month period, a longitudinal analysis of G-CSF, TNFα and IFN-gamma serum concentrations, burst activity of PMN, and expression of CD64 on the surface of PMN were performed by ELISA technique and flow cytometric analysis, respectively. It could be shown that peaks in G-CSF serum concentrations are followed by enhanced CD64 expression and increased burst activity of PMN in most patients with severe sepsis and septic shock. In addition, TNFα serum concentrations were elevated in most episodes, where G-CSF peaks were observed, whereas IFN-gamma serum concentrations were below the detection level in most episodes (Barth E et al., Peaks of endogenous G-CSF serum concentrations are followed by an increase in respiratory burst activity of granulocytes in patients with septic shock. Cytokine. 2002; 17(5): 275-84).

In addition, in a further study (Kragsbjerg P et al., Serum concentrations of interleukin-6, tumour necrosis factor-alpha, and C-reactive protein in patients undergoing major operations Eur J Surg. 1995; 161(1): 17-22) the kinetics of IL-6, TNFα and C-reactive protein after a surgical operation was investigated. It could be shown, that serum TNFα concentrations were low in all patients. Furthermore, the maximum serum concentrations of IL-6 and C-reactive protein after surgical operations were comparable to those in patients with sepsis. If IL-6 and C-reactive protein analyses are used in the diagnosis of infective complications, evaluation of the results should be related to the length of time between the operation and sampling, and to the clinical findings. The shorter period during which IL-6 was raised compared with C-reactive protein indicates that IL-6 may be a more useful marker of postoperative infective complications.

Thus, although many studies have been performed, it is still not clear whether low or high TNFα serum concentration is indicative for the risk of a postoperative sepsis. This also implies, that a single marker seems not to be reliable in order to to be used for such a risk prediction.

As mentioned above, a postoperative sepsis presumable arises from an immunodepression. Currently, a postoperative immunodepression can be monitored via the HLA-DR expression on monocytes by flowcytometry - the so far best standardized method to characterize cellular immune competence (Docke WD et al., Monitoring temporary immunodepression by flow cytometric measurement of monocytic HLA-DR expression: a multi-centre standardized study. Clin. Chem. 2005; 51:2341-7.) However, this method has several disadvantages. First of all there is a need of living cells, which are not stabile for a transport over 2h. Therefore, an experienced lab at the hospital is necessary. Thus, if a transport is not possible, and there is no central lab available, logistic problems will limit monitoring an immunodepression.

Thus, in view of the above, a more convenient and less time consuming approach for effective immune monitoring and improved treatment would be desirable. It is therefore an object of the present invention to define gene expression markers indicating postoperative immunodepression in order to provide a method that is suitable and effective in order to predict the risk of a postoperative sepsis in a human.

This object of the present invention, in one embodiment thereof, is solved by a method for the risk prediction of a postoperative sepsis in a human, comprising the steps of:
a) providing at least one biological sample from said human,
b) detecting the presence or a change in expression of at least one marker selected from the group consisting of perforin, CD3, and TNFα in said sample compared to a control, and
c) concluding from said presence or change in expression of said at least one marker on the risk of the development of a postoperative sepsis.

In another embodiment, the present invention also relates to a diagnostic kit for the risk prediction of a postoperative sepsis in a human, comprising materials, buffers and auxiliary agents and substances for performing the method according to the present invention. This kit can be used in a clinical environment and/or contains materials for high-throughput clinical serial testing.

Before the present invention is described in more detail below, it has to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references as cited herein are incorporated by reference in their entireties.

In the context of the present invention, a "biological sample" is any sample derived from one or more individuals that allows for detecting the presence or a change in expression of at least one marker selected from the group consisting of perforin, CD3, and TNFα in said sample. Examples of a biological sample are body fluids, such as blood, serum, urine, and the like, and/or samples containing cells, such as tissue samples. Preferred is a biological sample that is whole blood or serum. In addition, pools of samples coming from one or several different humans can be used as biological sample, in particular to distinguish subgroups of the presence or a change in expression of at least one marker selected from the group consisting of perforin, CD3, and TNFα.

In another preferred embodiment of the method according to the present invention, the biological sample is a whole blood sample. The use of a whole blood sample has several advantages. First of all a whole blood sample is easy to collect and about 2.5 ml whole blood are sufficient. Further, there is no cell separation necessary, and in addition, if the whole blood sample is collected in solutions with stabilizers for mRNA, e.g. PaxGene tubes, the whole blood sample is stable for hours at room temperature and for months if frozen.

In a preferred embodiment of the method according to the present invention, the at least one biological sample is obtained preoperative and/or postoperative. The biological sample obtained preoperative is preferably obtained on the same day of the surgery, but before the surgery. The biological sample obtained postoperative is preferably obtained on day one and/or two after surgery. Preferably, the at least one biological sample is obtained on day one and two after surgery.

The "expression" of a gene, or rather the protein (perforin, CD3 or TNFα) encoded by the gene, can be studied on three different levels: firstly, protein expression levels can be determined directly, secondly, mRNA transcription levels can be determined, and thirdly, the gene itself may be analysed for genetic modifications such as mutations, deletions, polymorphisms etc. influencing the expression of the gene product.

How to analyse the protein expression of a single gene is prior art. It usually requires an antibody specific for the gene product of interest. Appropriate technologies would be ELISA or immunohistochemistry. The analysis of the level of mRNA also has been described sufficiently. These days the gold standard is the reverse transcriptase PCR. How to analyse the gene itself is also known in the prior art. It usually requires sequencing, sometimes cloning, and/or database analyses.

All three markers according to the invention, namely perforin, CD3 and TNFα relate to genes and proteins that are known in the art. As mentioned above, TNFα as a archetypal acute inflammatory cytokines has been long known to be elevated in sepsis (Damas P et al, Tumor necrosis factor and interleukin-1 serum levels during severe sepsis in humans. 1989, Crit Care Med 17: 975-978). So far, it has been reported that serum TNFα is significantly higher in patients with sepsis as a complication than in those without sepsis (Nakae H et al, Involvement of IL-18 and soluble fas in patients with postoperative hepatic failure. 2003, Eur Surg Res. 35(2):61-6). In addition, perforin and CD3 are both known to be related to natural killer cells. Their involvement in sepsis, however, has not been clarified at all.

In a preferred embodiment of the method according to the present invention, a "control" is at least one biological sample obtained from a control-group (e.g. without a sepsis, particularly post-operative sepsis) and/or is at least one additional biological sample obtained from the same human. Preferably, the control is at least one biological sample obtained from a control-group and at least one additional biological sample obtained from the same human. In a further preferred embodiment of the method according to the present invention, the control-group are control-patients with the matching criteria selected from sex, age, type of surgery, and co-morbidities.

In another preferred embodiment of the method according to the present invention, the biological samples that are used as controls are obtained preoperative and/or postoperative.

In one preferred embodiment of the method according to the present invention, the control is at least one biological sample obtained from a control-group, and the at least one biological sample is obtained preoperative and/or postoperative. Preferably, one biological sample is obtained preoperative, and at least one additional biological sample is obtained postoperative. More preferably, one biological sample is obtained on the same day of surgery, but before the surgery and at least one additional biological sample is obtained on day one and/or two after surgery. Even more preferably, one biological sample is obtained on the same day of the surgery, but before the surgery, one biological sample is obtained on day one after surgery, and one biological sample is obtained on day two after surgery.

In another preferred embodiment of the method according to the present invention, the control is at least one additional biological sample from the same human, wherein said at least one additional biological sample is obtained preoperative and/or postoperative. Preferably, said additional biological sample should be obtained after surgery. Preferably, one biological sample is obtained preoperative and at least one additional biological sample is obtained postoperative. Preferably, one biological sample is obtained on the same day of the surgery, but before the surgery and at least one biological sample is obtained on day one and/or two after surgery. More preferably, one biological sample is obtained on the same day of the surgery, but before the surgery and one biological sample is obtained on day two after surgery. Even more preferably, the biological sample from the same human used as a control is obtained on the same day of the surgery, but before the surgery.

In a preferred embodiment of the method according to the present invention, all biological samples are taken at the same time period of the day in order to prevent circadian influences. Therefore, it is possible to take the biological samples for example in the morning or in the afternoon. The uptake of food should have no significant influence.

In one preferred embodiment of the method according to the present invention the presence or a change in expression of at least two, preferably three, predictive markers selected from the group consisting of perforin, CD3, and TNFα in said biological samples is detected. The combination in detection the presence or a change in expression of at least two, preferably three, predictive markers selected from the group consisting of perforin, CD3, and TNFα increases sensitivity, specificity, positive and negative predictive value.

In a further preferred embodiment of the method according to the present invention, the change in expression of at least two, preferably three, predictive markers selected from the group consisting of perforin, CD3, and TNFα in biological samples obtained pre- and/or post-operative is detected. Preferably, the change in expression of the three predictive markers perforin, CD3, and TNFα in biological samples obtained pre- and/or postoperative is detected.

In another preferred embodiment of the method according to the present invention, the change in expression of at least two, preferably three, predictive markers selected from the group consisting of Perforin, CD3, and TNFα is detected in biological samples obtained postoperative. Preferably, a postoperative decrease in expression of at least two, preferably three, predictive markers selected from the group consisting of perforin, CD3, and TNFα compared to their preoperative expression in the same human is detected. More preferably, a postoperative decrease in expression of the three, predictive markers perforin, CD3, and TNFα compared to their preoperative expression in the same human is detected.

In a further embodiment of the method according to the present invention the change in expression of at least two, preferably three, predictive markers selected from the group consisting of perforin, CD3, and TNFα is detected in biological samples obtained postoperative. Preferably, a postoperative decrease in expression of at least two, preferably three, predictive markers selected from the group consisting of Perforin, CD3, and TNFα compared to their postoperative expression in the control-group is detected. More preferably, a postoperative decrease in expression of the three, predictive markers Perforin, CD3, and TNFα compared to their prostoperative expression in the control-group is detected.

In one preferred embodiment of the method according to the present invention, the change in expression of the predictive marker and TNFα is detected in biological samples obtained preoperative. Preferably a preoperative decrease in expression of TNFα compared to its preoperative expression in the control-group is detected.

In another preferred embodiment of the method according to the present invention, the presence or a change in expression of at least one additional marker selected from the group of IL-1b, IL-8, IP-10, PF4, GRO1, MIP1a, CCR3, CD3, CD69, NKG2D, KLRD 1, granulysin, IL-6, IL-10, IL-18, SOCS3,TGFb, CD74, HLA-DR, HO-1, S100A8 in said biological samples is detected.

In a preferred embodiment of the method according to the present invention, the presence or a change in expression of the additional marker IL-1b in the biological samples is detected. Preferably, a postoperative decrease in expression of IL-1b compared to its postoperative expression in the control-group is detected. In addition, an impaired postoperative increase of expression in comparison to the expression in the control-group is detected.

In one embodiment of the method according to the present invention detecting the presence or a change in expression of the at least one predictive marker is performed using real time RT-PCR. For this, total RNA is isolated from the whole blood samples by standardized methods as known by a person skilled in the art and reversely transcribed into cDNA. Using specific primers and probes, the amount of cDNA is quantified by real-time RT-PCR, e.g. by Taqman.

In a preferred embodiment of the method according to the present invention the relative gene expression of the at least one predictive marker is measured by standardized methods as known by a person skilled in the art e.g. 2-ddCt-method, copies/microgram cDNA.

In one embodiment of the method according to the present invention concluding a risk of the development of a postoperative sepsis is indicated by a significant decrease of the expression of at least one predictive marker selected from the group consisting of perforin, CD3 and TNFα. In case of perforin, a significant decrease of the expression on day one and/or two after surgery in comparison to the preoperative expression as well as to the postoperative expression in the control-group is indicative for the risk for a postoperative sepsis. The same applies to CD3. In case of TNFα, a significant decrease of the expression on day one after surgery in comparison to the preoperative expression as well as a generally significant decrease of the expression in comparison to the expression in the control-group is indicative for the risk of a postoperative sepsis.

In a further embodiment of the method according to the present invention concluding a risk of the development of a postoperative sepsis is indicated by a significant decrease of the expression of the additional marker IL-1b. A significant decrease of the expression on day one and/or two after surgery accompanied by a changed postoperative increase of expression in comparison to the expression in the control-group is indicative for the risk of a postoperative sepsis.

In one embodiment of the method according to the present invention the significant decrease shows at least a p-value of p < 0.05. In the context of the present invention, a "p-value" shall mean the probability that a sample could have been drawn from the population(s) being tested (or that a more improbable sample could be drawn) given the assumption that the null hypothesis is true. A p-value of 0.05, for example, indicates that there would be only a 5% chance of drawing the sample being tested, if the null hypothesis was actually true. With a significant decrease is meant with respect to both the control-group or the preoperative expression of the marker.

In a further embodiment of the method according to the present invention concluding a risk of the development of a postoperative sepsis is indicated by a decrease of the expression of at least one additional marker selected from the group consisting of TGFb, CD3, CD74, HO-1, NKG2D, HLA-DRA, granulysin, KLRD1, CD69, CCR3, GRO-1, MIP1a, IL-8, PF4, and IP-10. In case the additional marker is selected from the group consisting of CD74, NKG2D, HLA-DRA, granulysin, KLRD1, CD69, CCR3, MIP1a, IL-8, and IP-10, a decrease of the expression on day one and/or two after surgery in comparison to the preoperative expression as well as in comparison to the postoperative expression in the control-group is indicative for the risk of a postoperative sepsis. In case the additional marker is selected from the group consisting of TGFb, HO-1, SOCS3, GRO-1, and PF4 a decrease of the pre- and postoperative expression in comparison to the pre- and postoperative expression in the control-group is indicative for the risk of a postoperative sepsis.

In a further embodiment of the method according to the present invention concluding a risk of the development of a postoperative sepsis is indicated by an increase of the expression of at least one additional marker selected from the group consisting of IL-10 and S100A8 on day one and/or two after surgery in comparison to the preoperative expression as well as to the postoperative expression in the control-group is indicative for the risk for a postoperative sepsis.

In another aspect thereof, the present invention relates to a diagnostic kit for the risk prediction of a postoperative sepsis in a human, comprising materials, buffers and auxiliary agents and substances for performing the method according to the present invention.

In yet another aspect thereof, the present invention relates to a method for preventing a post-operative sepsis in a human, comprising a method according to the present invention, and providing a suitable therapy to said human.

A suitable therapy according to the present invention comprises any therapy known by the person skilled in the art which is suitable in order to treat a postoperative sepsis. Such a suitable therapy comprises without any limitations a pre-emptive antibiotic therapy, and a pre-emptive immunostimulation, like GM-CSF, IFN-gamma (Docke WD et al., Monocyte deactivation in septic patients: restoration by IFN-gamma treatment. Nat Med. 1997; 3(6): 678-81).

The present invention will now be illustrated further on the basis of the examples with respect to the accompanying Figures, without being limited thereto. In the accompanying Figures,

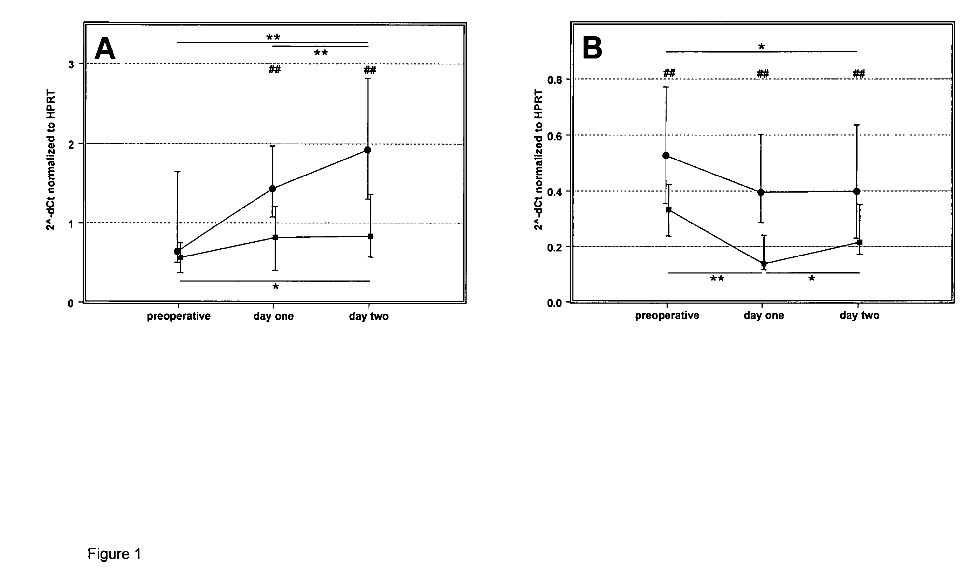

**Figure 1** shows a reduced mRNA expression of IL-1β and TNFα in patients developing sepsis. (A) The postoperative increase of IL-1b gene expression is impaired in the patients who developed sepsis. Following surgery, the control patients showed an increase on day one and day two after surgery that were highly significant on day two (p<0.002 compared to the preoperative value). While the gene expression is comparably in both groups before surgery, a significant reduction in mRNA quantity was found on both postoperative time points in the sepsis group compared to controls. (Displayed are medians and 25^{th} and 75^{th} percentiles. * = p<0.05 between two time points in a group (Wilcoxon); ** = p<0.01 between two time points in a group; # = p<0.05 between both groups at the indicated point of time (Mann-Whitney-U), ## = p<0.01 between both groups at the indicated point of time (Mann-Whitney-U). **(B)** Significant lower TNFα expression in the sepsis group over all three days. The mRNA quantity in the sepsis group is significantly lower than in the control group (p<0.006 preoperatively and on day two and p<0.00003 on the first day following surgery). While the gene expression rests stable in the control group, the mRNA is significantly reduced in the sepsis group relative to the preoperative value (p<0.005).

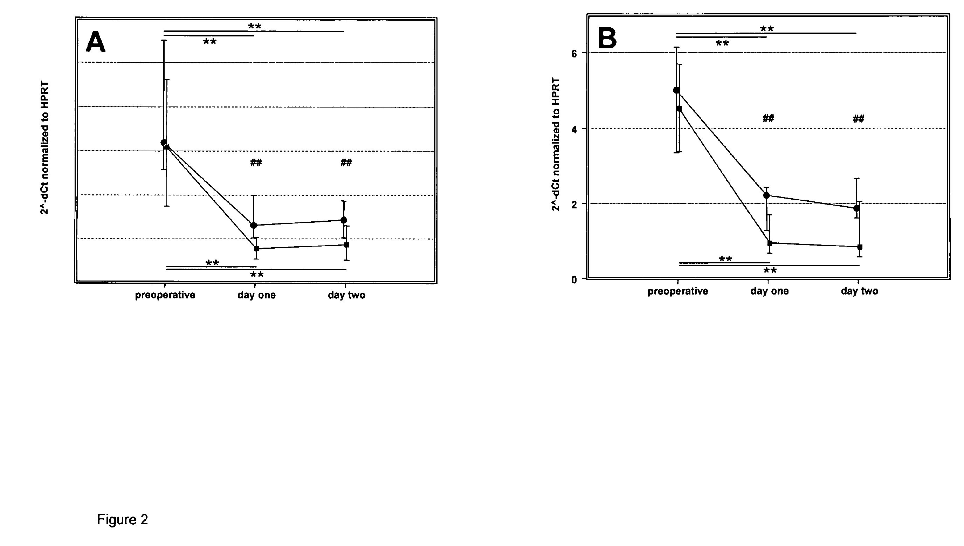

**Figure 2** shows a decreased expression of NK and T cell related gene products. (A) Perforin and **(B)** CD3 mRNA expression show a similar profile with a significant decrease in expression at the first day after surgery in both group and a lower expression level in the sepsis group. Expression reduction reaches its maximum at the first day after surgery and is significant in perforin and CD3. For explanation of symbols see legend to Figure 1A, above.

### EXAMPLES

### Retrospective case-control-study

In this case-control study 230 patients were enrolled. 20 patients with postoperative sepsis (3 sepsis and 17 severe sepsis/septic shock) were matched with 20 control-patients. The matching criteria were sex, age, type of surgery, and co-morbidities. (see Table 1):

**Table 1 Patients demographics. ASA (American Society of Anesthesiologists) is a score for the physical status of a patient, the ASA correlates with the risk of postoperative complications, morbidity and lethality, and is used widely.**

| | Sepsis | Control |
|---|---|---|
| Age (mean, range) | 64,75 (46-88) | 62,9 (45-80) |
| ASA (mean, range) | 2,85 (2-4) | 2,5 (2-3) |
| Main diagnosis | • Carcinoma of lung, esophagus, pancreas, co- | • Carcinoma of lung, pancreas, esophagus, colon |
| | lon, stomach | • Chronic cholangitis |
| | • Emphysema | • Hemoptysis |
| | • Bile duct leakage | • Pancreatitis |
| Surgery | Major abdominal and thoracic surgery | Major abdominal and thoracic surgery |

From each patient, three whole blood samples were taken using Paxgene® Blood RNA tubes (PreAnalytiX) on the morning before surgery and on two consecutive days.

The following selected genes (see Table 2) were analyzed and the relative gene expression was measured using the 2-ddCt-method as described in Livak et al. (Livak KJ, Schmittgen TD. Analysis of relative gene expression data using real-time quantitative PCR and the 2-DDCt method. Methods. 2001;25:402-408).

**Table 2**

| Chemokines | NKT-Cell related | Sepsis Cytokines | MHC-II associated | others |
|---|---|---|---|---|
| IL-8 | CD3 | TNFα | CD74 | HO-1 |
| IP-10 | CD69 | IL-1b | HLA-DRA | S100A8 |
| PF4 | Perforin | IL-6 | | |
| GRO-1 | NKG2D | IL-10 | | |
| MIP1a | KLRD1 | IL-18 | | |
| CCR3 | Granulysin | SOCS3 | | |
| | | TGFb | | |

It was found that preoperatively the gene expression of the transcription factor SOCS3 and the pro-inflammatory cytokine TNFα was significantly lower in the sepsis-group than in controls.

On the first day after surgery the relative gene expression of pro-inflammatory cytokines including TNFα, IL1b and MIP1a, T-cell/NK-cell associated KLRD1, perforin, CD3 as well as hemoxygenase (HO-1) and MHCII associated CD74 was reduced in the sepsis group compared to controls (p < 0.05). In addition, the gene expression of the anti-inflammatory cytokine TGFb showed no differences between the groups, and IL-10 expression was higher in the septic group (not significant). Multivariate statistical analysis was performed using a Non-parametric Analysis of Longitudinal Data (SAS-Macro by Brunner, University Goettingen, Germany). For the comparison between the case and the control group, we used the Mann-Whitney-U test. For selected genes the Bonferoni correction method for repeated testing was applied in order to calculate the risk prediction of sepsis.

In signal detection theory, a receiver operating characteristic (ROC), also receiver operating curve, is a graphical plot of the sensitivity vs. (1 - specificity) for a binary classifier system as its discrimination threshold is varied. The ROC can also be represented equivalently by plotting the fraction of true positives (TP) vs. the fraction of false positives (FP). The best possible prediction method would yield a graph that was a point in the upper left corner of the ROC space, i.e. 100% sensitivity (all true positives are found) and 100% specificity (no false positives are found). A completely random predictor would give a straight line at an angle of 45 degrees from the horizontal, from bottom left to top right (the so-called 'line of no-discrimination'): this is because, as the threshold is raised, equal numbers of true and false positives would be let in. Results below this no-discrimination line would suggest a detector that gave wrong results consistently, and could therefore be simply used to make a detector that gave useful results by inverting its decisions. Using said ROC-curve analysis the inventors identified three genes of special interest for prediction of sepsis. The combination of perforin, CD3 and TNFα (AUC: 0.81, 0.84, 0.86, respectively) was able to predict postoperative sepsis with a sensitivity of 75% and a specificity of 100% at the first day after surgery (see Table 3)

**Table 2**

| | | Sepsis | | Sepsis | | Total | |
|---|---|---|---|---|---|---|---|
| | | | 0 | | 1 | | |
| Test 0 | number | | 20 | | 5 | | 25 |
| | % of Sepsis | | 100% | | 25% | | 62.5% |
| Test 1 | number | | 0 | | 15 | | 15 |
| | % of Sepsis | | 0% | | 75% | | 37.5% |
| Total | | | 20 | | 20 | | 40 |
| | | | 100% | | 100% | | 100% |

| | | | | | | | |
|---|---|---|---|---|---|---|---|
| P< 0,001 sensitivity: 75% Specificity: 100% | | | | | | | |

The significantly lower expression of inflammatory cytokines such as TNFα and IL1b leads to the conclusion that patients with postoperative sepsis suffered from a more pronounced immunodepression after surgery, even though the gene expression of anti-inflammatory cytokines remained unchanged. This emphasises the influence of the postoperative immune status on the occurrence of complications. The presented pilot-study showed that gene expression based tests have the power to identify patients at risk before the occurrence of clinical symptoms.

## Claims

1. A method for the risk prediction of a postoperative sepsis in a human, comprising the steps of:
a) providing at least one biological sample from said human,
b) detecting the presence or a change in expression of at least one marker selected from the group consisting of Perforin, CD3 and TNFα in said sample compared to a control, and
c) concluding from said presence or change in expression of said at least one marker on the risk of the development of a postoperative sepsis.

2. The method according to claim 1, wherein said at least one biological sample is obtained preoperative and/or postoperative.

3. The method according to claim 2, wherein said biological sample is a whole blood sample.

4. The method according to any of claims 1 to 3, wherein said control is at least one biological sample obtained from a control-group and/or is at least one additional biological sample obtained from the same human.

5. The method according to claim 4, wherein said control-group are control-patients having the matching criteria sex, age, type of surgery, and co-morbidities.

6. The method according to claim 4 or 5, wherein said biological samples used as controls are obtained preoperative and/or postoperative.

7. The method according to any of claims 1 to 6, wherein all biological samples are taken during the same time period or at the same time point of the day.

8. The method according to any of claims 1 to 7, wherein the presence or a change in expression of at least two, preferably three, predictive markers selected from the group consisting of Perforin, CD3 and TNFα in said biological samples is detected.

9. The method according to any of claims 1 to 8, wherein the presence or a change in expression of at least one additional marker selected from the group of IL1b, IL-8, IP-10, PF4, GRO1, MIP1a, CCR3, CD3, CD69, NKG2D, KLRD1, Granulysin, IL-6, IL-10, IL-18, SOCS3,TGFb, CD74, HLA-DR, HO-1, and S100A8 is detected in said biological sample(s).

10. The method according to claim 9, wherein the presence or a change in expression of at the additional marker IL1b is detected in said biological sample(s).

11. The method according to any of claims 1 to 10, wherein detecting the presence or a change in expression of said at least one predictive marker is performed using real time RT-PCR.

12. The method according to any of claims 1 to 11, wherein a significant decrease of the expression of at least one predictive marker selected from the group consisting of Perforin, CD3 and TNFα is indicative for the risk for a postoperative sepsis.

13. The method according to claim 12, wherein said significant decrease has a p of at least < 0.05.

14. A diagnostic kit for the risk prediction of a postoperative sepsis in a human, comprising materials, buffers and auxiliary agents and substances for performing the method according to any of claims 1 to 13.

15. A method for preventing a postoperative sepsis in a human, comprising a method according to any of claims 1 to 13, and providing a suitable therapy to said human.
